# EUROPEAN PATENT SPECIFICATION

(11) **EP 3 024 516 B1**
(45) Date of publication and mention of the grant of the patent: **05.01.2022**
(21) Application number: 14750604.2
(22) Date of filing: 25.07.2014
(51) Int. Cl.: A61M 5/20, A61M 5/24, A61M 5/315

(54) **INJECTION DEVICES**
INJEKTIONSVORRICHTUNGEN
DISPOSITIFS D'INJECTION

(30) Priority: 25.07.2013 GB 201313287
(43) Date of publication of application: 01.06.2016
(73) Proprietor: Owen Mumford Limited, Oxfordshire OX20 1TU (GB)
(72) Inventor: COWE, Toby, Oxford Oxfordshire OX20 1TU (GB)
(74) Representative: Lind, Robert
(86) International application number: PCT/GB2014/052276
(87) International publication number: WO 2015/011488

(56) References cited:
- WO-A2-2012/049468
- US-A1- 2011 144 594
- US-A1- 2013 172 822
- None

## Description

### FIELD OF THE INVENTION

This invention relates to injection devices and in particular, but not exclusively to a pen injector device.

### BACKGROUND OF THE INVENTION

Injection devices are used for the convenient administration of medicaments. For example, pen injectors may be used for providing a single metered dose of a medicament, such as Epinephrine, in an emergency or for providing regular metered doses of a medicament, such as insulin.

One such injector device has been proposed in the applicants co-pending International Patent Application No. PCT/GB2011/051950. The injector device of this patent
application includes an elongate housing; a syringe disposed in said housing and having an internal piston to express a dose from a needle at its front end; a trigger device for, in use, activating the internal piston. A cap is provided that, in a fitted configuration, fits over at least part of the elongate housing and prevents activation of the trigger device.

One particular advantage of this prior art injector device is that the overall size of the injector may be reduced since, in the forward (delivery) position, the drive spring at least partially surrounds the plunger and extends into the inner of the syringe. It is desirable for pen injectors to be of a compact form so that they can be carried around and used unobtrusively. Further
compact injectors may be simple to manufacture, assemble and use with consequent savings in manufacturing and assembly costs, and a lower environmental impact.

In some injectors it may be necessary use high force springs (for example due to high viscosity drugs or small needle diameters). As such there may be a need for an injector having a more resilient mechanism. Additionally, the applicant has now recognised that there may be a risk that the spring could catch on the neck of the aperture or within a spring coil at the rear of the syringe which could delay or prevent firing of the injection mechanism.

Embodiments of the present invention are intended to address at least some of the abovementioned problems.

US2011/144594 discloses a safety member for use with an injection device.

US2013/172822 discloses an auto-injector for administering a dose of a liquid medicament with a delay mechanism for slowing down motion of a retraction sleeve.

WO2012/049468 discloses an injection device that comprises a shroud element, to shroud the needle after use, and a latch movable between a latched state, in which it restricts rearward movement of the shroud element, and a release state. The latch is moved to its latch state by a magnetic force.

### SUMMARY OF THE INVENTION

Accordingly, in one aspect, this invention provides an injection device as claimed in claim 1.

The latch member may be axially fixed. The radial displacement of the latch member may be provided by a portion of the latch member flexing or hinging. For example the latch member may resiliently flex.

The plunger may have a forward end sized and shaped to be received within the internal bore of a syringe. The actuation mechanism may provide a forward biasing force on the plunger. The actuation mechanism may comprise at least one biasing member (for example a compression spring).

At least a portion of the trigger may be disposed radially outside of the latch member.

The trigger may be slidably mounted within the housing. The trigger may be biased or held against the activation direction. For example, the trigger may be rearwardly biased and may, for example, be urged forwards against said bias to activate the injector device. The trigger device can be biased by means of at least one sprung member. The at least one sprung member can include a cam surface which, in use when sliding in the activation direction, rides over an abutment inside the housing. The trigger may conveniently be an axially moveable button (for example provided at an end of the injection device).

The trigger may comprise a forwardly extending portion which is disposed radially outside of the latch member.

The forwardly extending portion may substantially surround the latch member. For example the forwardly extending portion of the trigger may be generally cylindrical.

One of the latch member and the trigger may be provided with a substantially radially projecting member which is arranged to engage an opposing surface of the other of the trigger and latch member. At least one pair of radially opposed projecting members may be provided. The, or each, radially projecting member may comprise a boss. When the trigger is in the primed position the radially projecting member may abut the opposing surface to prevent radial movement of the latch member. When the trigger is in the release position the radially projecting member may move clear of the opposing surface. For example, the surface of the trigger or latch member which opposes the radially projecting member may comprise at least one cut out sized and shaped to receive the at least one outwardly projecting member. The cut out may be arranged to be moved into alignment with the boss when the trigger is in the release position.

The latch member may be provided with at least one radially outwardly projecting boss on an exterior surface. The at least one radially outwardly projecting boss may be arranged to abut an interior surface of the trigger when the trigger is in the primed position.

The trigger may comprise at least one cut out sized and shaped to receive the at least one outwardly projecting boss. The cut out may be arranged to be moved into alignment with the boss when the trigger is in the release position. The cut out may, for example, be provided on the forwardly projecting portion of the trigger.

It will be appreciated that prior to the movement of the trigger to the relaease position the radially projecting member or boss may abut the opposing surface so as to inhibit radially expansion of the latch member. Upon movement of the trigger to the release position the member or boss may move into alignment such that the boss or member and surface no longer abut and the latch is free to expand. Advantageously, such an arrangement can be used to enable more accurate control of the point of release of the plunger from the latch member. For example, the alignment of a member or boss with a corresponding cut out may provide an arrangement where the latch will only release the plunger upon full alignment of the boss or member and cut-out.

The enlarged head may comprise a rearwardly outwardly tapering surface. For example the outwardly tapering surface may be substantially frustoconical. The tapering surface may act to cam the flexible portions of the latch member outward as the plunger passes through the aperture.

A rear portion of the enlarged head may comprise a rearwardly inwardly tapering surface. The inwardly tapering surface may be frustoconical. The inwardly tapering rear portion may help prevent the plunger catching as it exits the aperture and may allow any return flexure of the latch member to help push the plunger through the aperture.

Forward movement of the trigger towards the actuation position may be arranged to engage the rearward end of the plunger such that the trigger may physically urge the plunger through the aperture. Thus, the trigger may be arranged to aid the release of the plunger from the latch member.

This arrangement may, for example, be particularly advantageous in injectors utilising high stiffness springs and is considered novel and inventive in its own right. Thus, according to a further aspect the invention provides an injector comprising:
a plunger for expressing medicament from a syringe;
an actuation mechanism arranged in use to provide a forward biasing force to urge the plunger from a first rearward position to a second forward position to express a dose from the syringe;
a latch member arranged to retain the plunger against the force of the actuation mechanism; and
a trigger for releasing the plunger from the latch member; and wherein the trigger is further arranged to physically engage (for example abut) a portion of the plunger to reinforce the forward biasing force of the actuation member to overcome the retaining force of the latch member.

The trigger may reinforce the forward biasing force by being arranged to transmit a forward axial force on the plunger during depression of the trigger.

This aspect may be combined with any of the features refered to above with respect to the first aspect of the invention.

The trigger may comprise an axially moveable button disposed at the rear of the injector and comprising a rearward facing surface for engagement by a user and an opposing forward facing surface and wherein the forward facing surface is arranged to engage the rearward portion (for example an end face) of the plunger as the trigger is moved from the primed position to the release position.

The trigger and plunger may be provided with interconnecting features. For example,the forward facing surface may be provided with an inwardly extending projection for engaging rearward tip of the plunger. The tip of the plunger may be provided with a recess or depression sized and shaped to receive the projection.

The latch member is formed integrally with the housing. For example, the latch member may be an inner housing member provided within a rearward portion of the housing.

The latch member may comprise an axially split member. The latch member comprises a radially segmented member, for example a cylindrical member. The at least one axially extending slot may be arranged to allow the latch member to flex radially. Advantageously, the stiffness of the latch member (and the resulting force required to release the plunger) may be easily tailored for example by adjusting any of the material stiffness, the dimensions of the slot, the number of slots, the latch face angle and the thickness of the walls of the latch member.

Whilst the invention has been described above, it extends to any inventive combination set out above, or in the following description or drawings.

### BRIEF DESCRIPTION OF THE DRAWINGS

The invention may be performed in various ways, and an embodiment thereof will now be described by way of example only, reference being made to the accompanying drawings, in which:
Figure 1 is an exploded view of the first embodiment of an autoinjector in accordance with this invention;
Figure 2(a) and 2(b) are respective side and top section views of the autoinjector of Figure 1 assembled and prior to use;
Figure 3 is a three dimensional view of the latch member of the autoinjector of Figure 1; and
Figures 4(a) to (d) are partial cross sectional views of the rearward portion of the autoinjector of Figures 1 and 2 taken through the line of the boses on the latch member showing the activation sequence.

### DETAILED DESCRIPTION OF EMBODIMENTS

Front as used herein will be understood to refer to the end of the injector assembly (or components thereof) which, in use, are closest to the delivery needle delivery end of the injector (i.e. the end which is pointed at the skin). Rear as used herein will be understood to refer to the end of the pen injector assembly (or components thereof) which, in use, are furthest from the needle delivery end of the injector (i.e. the end which is pointed away from the skin). Forward and rearward will, likewise, be understood to refer to the directions orientated towards the front and rear of the injector assembly.

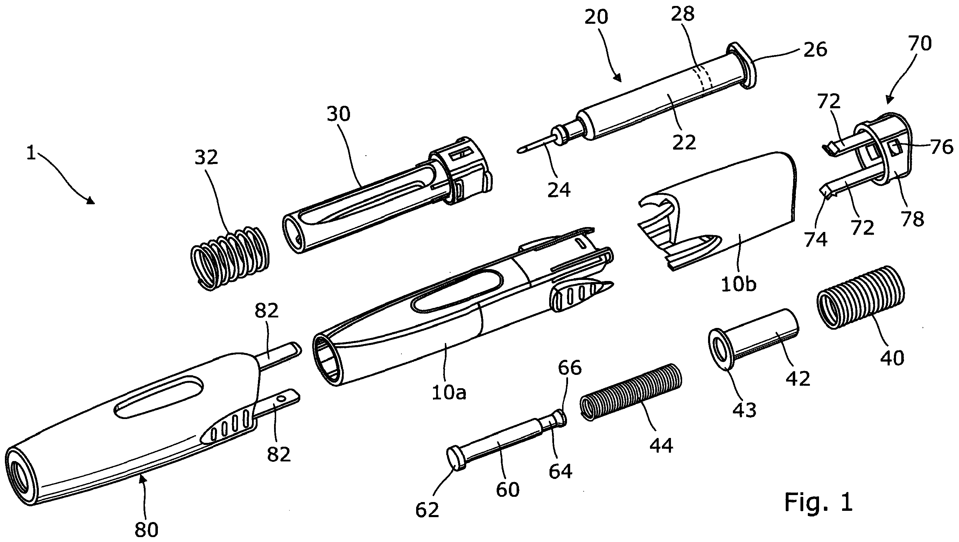

Referring initially to Figures 1 to 3, the autoinjector 1 comprises an outer housing 10 of cylindrical form in the bore of which is disposed a syringe 20. The syringe is formed of front 10a and rear 10b housing portions (split for convenience of manufacture). The syringe is of known form with a barrel 22, a needle 24 extending from the forward end, and a flange 26 at its rear end. A medicament is contained within the syringe and can be expressed through the needle by a piston 28 inside the barrel. The syringe is supported and surrounded by a carrier assembly 30. A biasing spring 32 is provided which, in the assembled state, biases the carrier 30 rearwardly such that the needle 24 of the syringe 20 is shrouded within the housing 10.

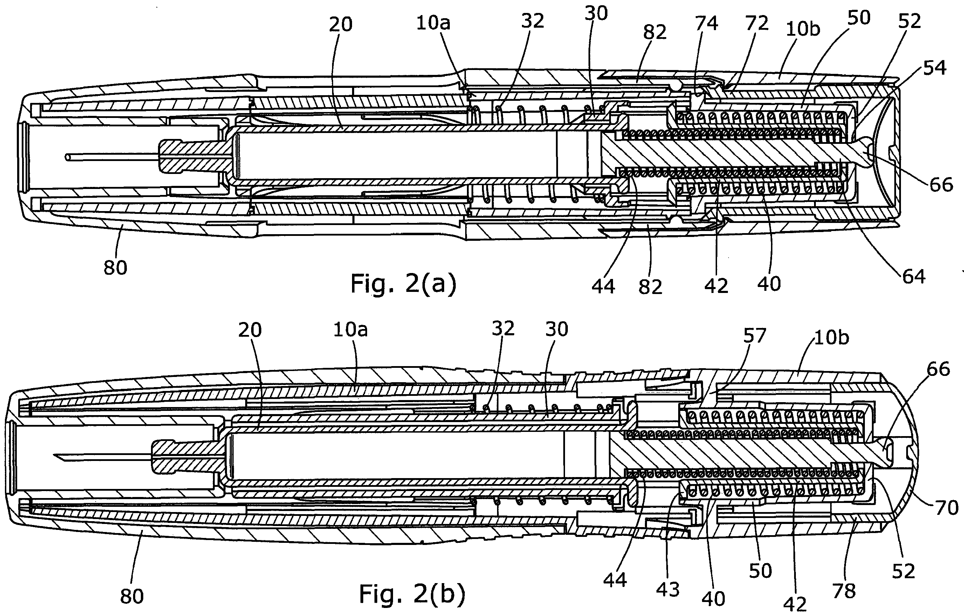

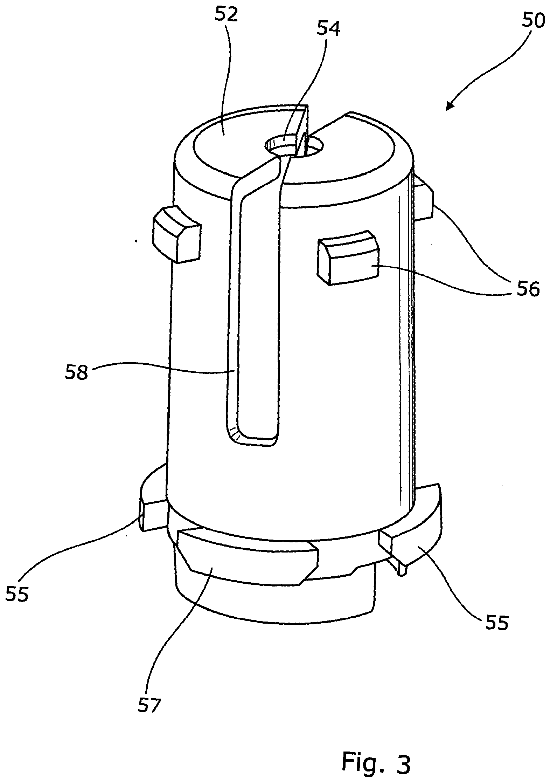

In the rear of the housing 10b is provided an actuation mechanism which is arranged such that when released it urges the plunger forward relative to the housing. The actuation mechanism includes a first, outer, spring 40 which acts between the front face of a transverse inner wall 52 which is formed as part of a latch member 50 (which is best seen in figure 3 and described in further detail below) at the rear of the housing 10 and a forward flange 43 of a top hat-shaped intermediate member 42. A second, inner, spring 44 is received within a cylindrical part of the intermediate member 42 and acts between an inner face of the rear end wall thereof and a circumferential rib 62 on the forward part of a plunger 60. At the rear end, the plunger has a reduced diameter neck 64 with an enlarged head 66 (which will be described in further detail below). In the initial position shown in Figure 2, the neck 64 is located within an aperture 54 in the transverse wall 52 of the housing latch member 50. The enlarged head 66 retains the plunger in position within the aperture 54.

Projecting rearwardly from the rear end of the housing is a captive axially slideable trigger button 70 movable against a rearward bias from the initial position, shown for example in Figure 2. The trigger button 70 includes a forwardly extending portion 78 which has a generally cylindrical profile. As best seen in the cross section of figure 2, the forwardly extending portion fits within the rear housing portion 10b and radially surrounds the rear portion of the latch member 50. The forwardly extending portion 78 is provided with a plurality of cut-outs 76 (which are sized and dimensioned to receive bosses 56, shown in figue 3, of the latch member as will be described below). The trigger button 70 is held in place by two integral forwardly extending arms 72 with cam surfaces 74 which ride over respective abutments 55 inside the rear of the housing. However, initially, forward movement of the trigger button is prevented by means of two rearwardly extending locking arms 82 which extend back from the rear end of a cap 80. The arrangement and function of the cap is substantially as described in in the applicants co-pending International Patent Application No. PCT/GB2011/051950.

The latch member 50 is shown in isolation in figure 3 (although it will be appreciated that in someembodiments the latch member 50 may be formed as an inner portion of the rear housing 10b which is spaced apart from and within the housing 10b). The latch member 50 has a generally cylindrical body which is segmented at its rear end by a pair of opposing axially extending slots 58 such that the rear portion of the body provides two opposing segments (although it will be appreciated that further slots may be provided to define a greater number of segments). The slots 58 join at the transverse inner wall 52 at the rear of the latch member and are conjoined with the aperture 54. As such the aperture 54 may be considered to be a split aperture. Thus, it will be appreciated that the slot 58 enables the segments of the latch member to resiliently flex radially outwardly to enable the aperture 54 to be expanded. Proximal to the rearward end of the latch member 50 radially outwardly projecting bosses 56 are provided spaced circumferentially around the latch member. For example, the boses may be arranged in pairs which are substantially radially opposed (so as to constrain in the radial direction in which they oppose one another), The forward end of the latch member 50 is provided with two outwardly radially projecting abutments 55 over which the arms 72 ride when the button is depressed. It may be noted that figure 3 appears to show 4 such abutments but the other outward projections 57 are, in fact, connecting portions which extend between the latch member 50 and the rear housing 10b.

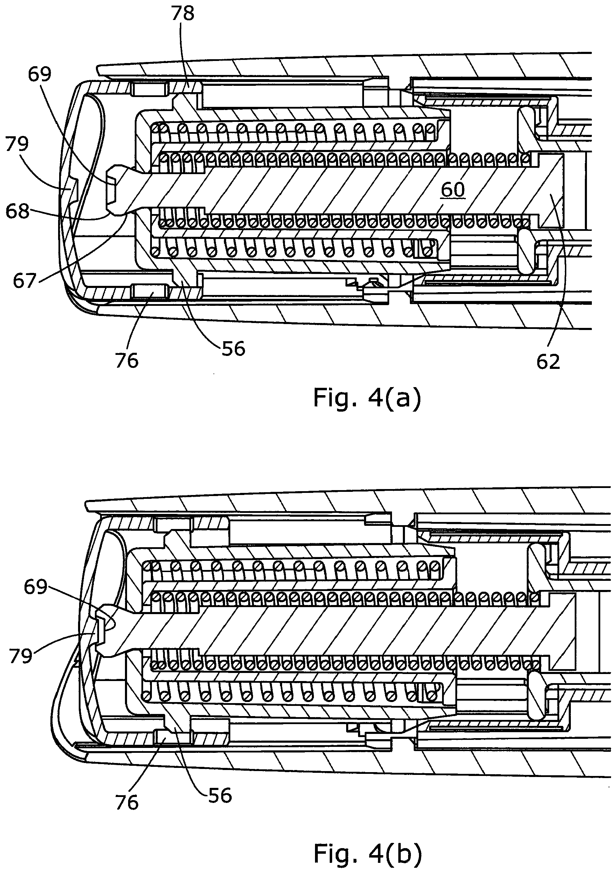

In operation, the user pulls the cap 80 off forwardly which arms the device by rendering the trigger button 70 operational (and which may typically also remove a needle shield, not shown, from the syringe). In this primed state, as shown in figure 4(a), the trigger button 70 is biased to its rearward position and the reduced diameter neck 64 at the rear of the plunger 60 is positioned within the aperture 54 of the latch member 50. The plunger 60 is subject to a biasing force in the forward direction from the springs 40, 44 of the actuation mechanism but is held captive in its rearward position as the enlarged head 66 cannot pass through the aperture 54 without the segmented sections of the latch member 50 being flexed radially outwardly. Such outward movement is prevented since the bosses 56 abut the inner surface of the forwardly extending portion 78 of the trigger button 70.

Following removal of the cap, the user offers the injection device up to the injection site and presses the trigger button 70. The trigger button slides forwardly from the position shown in figure 4(a) to the position shown in figure 4(b). As the button 70 moves forward the cut outs 76 in the forward portion of the trigger button 70 are brought into alignment with the bosses 56 of the latch member 50. In this position (shown in figure 4(b)) the latch member is no longer constrained against radial expansion by the trigger button. It will be noted that in this position the forwardly projecting member 79 provided on the internal surface of the trigger button 70 which may engage the rearward end of the plunger. The rearward end of the plunger 60 may be provided with correspondingly profiled recess 69 into which the member 79 may locate. This enables a portion of the actuating force provided by the user to be directly transferred into a forward force upon the plunger 60. This arrangement may be particularly useful in providing a "fail safe" arrangement in which the member 79 of the trigger button 70 only engages the plunger recess 69 if the plunger does not immediately release upon the trigger moving towards the forward position. In other words, the plunger may normally be expected to be released by the driving force of the springs 40, 42 and the member 79 may provide a reinforcing action in the event of any stiction in the syringe or mechanism or the need to design the latch mechanism to be very resilient to forward movement of the springs (as may be required when high force springs are used for high viscosity drugs or small needle diameters).

Thus, under the forward driving force of the springs 40, 42 of the actuation mechanism (possibly reinforced by direct force from the trigger button 70 on the end of the plunger 60) the enlarged head 66 may be forced through the aperture 54 by flexing the segments of the latch member 50 radially outwardly. Advantageously, the enlarged head 66 is profiled along its axial length to assist its transition through the aperture 54. In particular, the forward portion 67 of the enlarged head 66 has a rearwardly outwardly tapering profile which acts to cam the segments of the latch 50 radially outwardly as the plunger moves forward. The rearward portion 68 of the enlarged head has a rearwardly inwardly tapering profile which helps to ensure that the head 66 will smoothly leave the aperture 54 without being caught by any resilient spring back of the latch member 50 (which would instead only act to cam the head through the aperture).

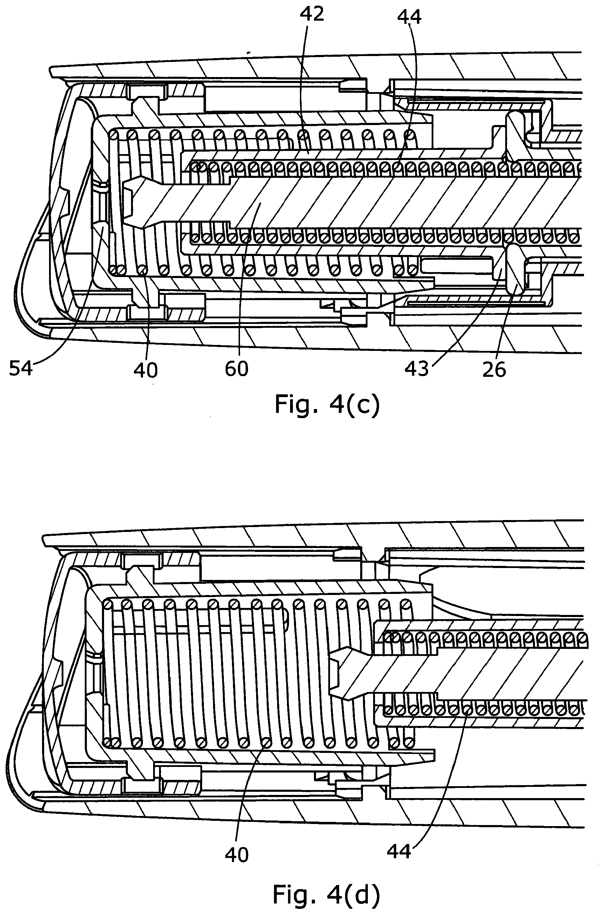

Once the plunger is released (as shown in figures 4(c) and 4(d)), the first spring 40 expands to extend the syringe 20 so that the needle 24 penetrates the flesh. Initially, the second spring 44 typically remains substantially fully compressed, with the plunger 28 bearing against the piston 20 within the syringe but not moving it relative to the syringe (for example due to the compressibility of the medicament therein). Once the syringe has been moved to its fully forward position the second spring 44 may act between the a circumferential rib 62 on the forward part of a plunger 60 and the intermediate member 42 to drive the plunger 28 forward and dispense the medicament through the needle 24. It may be noted that during this second phase the second spring 44 extends into the barrel 22 of the syringe 20 along with the plunger 60, this is advantageous in reducing the overall size of the injector device. The taper leading to the enlarged latching head of the plunger ensures that the plunger does not catch on the second spring at this point.

Although the invention has been described above with reference to a preferred embodiments, it will be appreciated that various changes or modifications may be made without departing from the scope of the invention as defined in the appended claims. For example, the latch member may be split into any convenient number of segments and, for example at least one boss may be provided on each section. Alternatively or additionally, the bosses may be omitted from the latch member and the trigger may simply abut a portion of the latch surface in the primed position. Alternatively, the boses and cut-outs may for example be reversed without substrantially altering their function of blocking/freeing the radial movement of the latch member. Thus in some embodiments inwardly extending projections (such as boses) could be formed on the trigger which abut a surface of the latch member in the primed position and move out of aligment (for example by moving into alignment with a cut-out on outer surface of the latch) with in the release position. In some embodiments the device may be a single use device and, for example, the syringe may be integrally formed with the housing.

## Claims

1. An injection device (1) comprising:
a plunger (60) for expressing medicament from a syringe (20), the plunger being provided with an enlarged head (66) at a rearward end;
an actuation mechanism arranged in use to provide a biasing force to urge the plunger (60) from a first rearward position to a second forward position to express a dose of medicament;
a latch member (50) comprising an aperture (54) through which the rearward end of the plunger extends in its rearward position so as to be retained against the force of the actuation mechanism; and
a trigger (70) for releasing the plunger (60) from the latch member (50);
one of the latch member and the trigger is provided with a substantially radially projecting member (56) which is arranged to engage an opposing surface (78) of the other of the trigger and the latch member; and
**characterised in that**
the latch member (50) is arranged to at least partially radially displace and is radially segmented by at least a pair of opposing axially extending slots (58) to provide two or more opposing segments; and
the aperture (54) is provided in a transverse wall (52) at the rear of the latch member with the slots (58) joining at the transverse wall (52) and the slots being conjoined with the aperture (54)
such that the aperture (54) expands when the latch member is radially displaced to release the enlarged head (66) of the plunger; and
wherein the trigger (70) is moveable between
a primed position in which the member (56) and opposing surface (78) prevent radial displacement of the latch member and
a release position in which the radially projecting member (56) moves clear of the opposing surface to allow displacement of the latch.

2. An injector as claimed in claim 1 wherein, at least a portion of the trigger (70) is disposed radially outside of the latch member (50).

3. An injector as claimed in claim 2 wherein, the trigger (70) is slidably mounted within a housing (10) of the injector and comprises a forwardly extending portion (78) which is disposed radially outside of the latch member (50).

4. An injector as claimed in claim 3 wherein, the trigger (70) comprises an axially moveable button.

5. An injector as claimed in claim 3 or 4 wherein, the forwardly extending portion (78) substantially surrounds the latch member (50).

6. An injector as claimed in any preceding claim, wherein the outwardly projecting member comprises at least one radially outwardly projecting boss (56) provided on an exterior surface of the latch member and the opposing surface comprises an interior surface of the trigger (70) which the boss (56) abuts when the trigger is in the primed position.

7. An injector as claimed in claim 6, wherein the trigger (70) comprises at least one cut out (76) sized and shaped to receive the at least one outwardly projecting boss (56) and wherein the cut out is moved into alignment with the boss when the trigger is in the release position.

8. An injector as claimed in any preceding claim, wherein the enlarged head (66) comprises a rearwardly outwardly tapering surface (67) which acts to cam the flexible portions (52) of the latch member (50) outward as the plunger (60) passes through the aperture (54).

9. An injector as claimed in claim 8, wherein a rear portion of the enlarged head (66) comprises a rearwardly inwardly tapering surface (68).

10. An injector as claimed in any preceding claim, wherein the trigger (70) comprises an axial moveable button disposed at the rear of the injector (1) and comprising a rearward facing surface for engagement by a user and an opposing forward facing surface and wherein the forward facing surface is arranged to engage the rearward portion of the plunger as the trigger is moved from the primed position to the release position to reinforce the forward biasing force of the actuation member to overcome the retaining force of the latch member.

11. An injector as claimed in claim 10, wherein the trigger is arranged such that a portion of the actuating force provided by the user on the trigger (70) is directly transferred into a forward force upon the plunger (60).

12. An injector as claimed in claim 10 or 11, wherein the forward facing surface is provided with an inwardly extending projection (79) for engaging a rearward tip of the plunger.

13. An injector as claimed in claim 12, wherein the tip of the plunger is provided with a recess or depression (69) sized and shaped to receive the projection (79).

14. An injector as claimed in any preceding claim, wherein the latch member (50) is formed integrally with the housing.

15. An injector as claimed in any preceding claim, wherein the latch member (50) comprises a radially segmented cylindrical member.

## Patentansprüche

1. Einspritzvorrichtung (1), die Folgendes umfasst:
einen Kolben (60) zum Herausdrücken eines Medikaments aus einer Spritze (20), wobei der Kolben an einem hinteren Ende mit einem vergrößerten Kopf (66) versehen ist,
einen Betätigungsmechanismus, der bei Anwendung dafür angeordnet ist, eine Vorspannkraft bereitzustellen, um den Kolben (60) von einer ersten hinteren Stellung zu einer zweiten vorderen Stellung zu drängen, um eine Dosis eines Medikaments herauszudrücken,
ein Klinkenelement (50), das eine Öffnung (54) umfasst, durch die sich das hintere Ende des Kolbens in seiner hinteren Stellung erstreckt, so dass er gegen die Kraft des Betätigungsmechanismus zurückgehalten wird, und
einen Auslöser (70) zum Lösen des Kolbens (60) von dem Klinkenelement (50),
wobei das eine von dem Klinkenelement und dem Auslöser mit einem im Wesentlichen in Radialrichtung vorspringenden Element (56) versehen ist, das dafür angeordnet ist, eine gegenüberliegende Fläche (78) des anderen von dem Auslöser und dem Klinkenelement in Eingriff zu nehmen, und
**dadurch gekennzeichnet, dass**
das Klinkenelement (50) angeordnet ist, um sich mindestens teilweise in Radialrichtung zu verschieben, und durch mindestens ein Paar von gegenüberliegenden sich in Axialrichtung erstreckenden Schlitzen (58) in Radialrichtung segmentiert ist, um zwei oder mehr gegenüberliegende Segmente bereitzustellen, und
die Öffnung (54) in einer Querwand (52) an der Rückseite des Klinkenelements bereitgestellt wird, wobei sich die Schlitze (58) an der Querwand (52) vereinen und die Schlitze mit der Öffnung (54) verbunden sind,
derart, dass sich die Öffnung (54) ausdehnt, wenn das Klinkenelement in Radialrichtung verschoben wird, um den vergrößerten Kopf (66) des Kolbens freizugeben, und
wobei der Auslöser (70) beweglich ist zwischen
einer vorbereiteten Stellung, in der das Element (56) und die gegenüberliegende Fläche (78) eine radiale Verschiebung des Klinkenelements verhindern, und
einer Freigabestellung, in der sich das in Radialrichtung vorspringende Element (56) von der gegenüberliegenden Fläche weg bewegt, um eine Verschiebung der Klinke zu ermöglichen.

2. Einspritzvorrichtung nach Anspruch 1, wobei mindestens ein Abschnitt des Auslösers (70) in Radialrichtung außerhalb des Klinkenelements (50) angeordnet ist.

3. Einspritzvorrichtung nach Anspruch 2, wobei der Auslöser (70) verschiebbar innerhalb eines Gehäuses (10) der Injektionsvorrichtung angebracht ist und einen sich nach vorn erstreckenden Abschnitt (78) umfasst, der in Radialrichtung außerhalb des Klinkenelements (50) angeordnet ist.

4. Einspritzvorrichtung nach Anspruch 3, wobei der Auslöser (70) einen in Axialrichtung beweglichen Knopf umfasst.

5. Einspritzvorrichtung nach Anspruch 3 oder 4, wobei der sich nach vorn erstreckende Abschnitt (78) das Klinkenelement (50) im Wesentlichen umgibt.

6. Einspritzvorrichtung nach einem der vorhergehenden Ansprüche, wobei das nach außen vorspringende Element mindestens einen in Radialrichtung nach außen vorspringenden Buckel (56) umfasst, der auf einer Außenfläche des Klinkenelements bereitgestellt wird, und die gegenüberliegende Fläche eine Innenfläche des Auslösers (70) umfasst, an die der Buckel (56) anstößt, wenn sich der Auslöser in der vorbereiteten Stellung befindet.

7. Einspritzvorrichtung nach Anspruch 6, wobei der Auslöser (70) mindestens einen Ausschnitt (76) umfasst, der dafür bemessen und geformt ist, den mindestens einen nach außen vorspringenden Buckel (56) aufzunehmen, und wobei der Ausschnitt in Ausrichtung mit dem Buckel bewegt wird, wenn sich der Auslöser in der Freigabestellung befindet.

8. Einspritzvorrichtung nach einem der vorhergehenden Ansprüche, wobei der vergrößerte Kopf (66) eine sich nach hinten und nach unten verjüngende Fläche (67) umfasst, die wirkt, um die flexiblen Abschnitte (52) des Klinkenelements (50) nach außen zu kanten, wenn der Kolben (60) durch die Öffnung (54) hindurchgeht.

9. Einspritzvorrichtung nach Anspruch 8, wobei ein hinterer Abschnitt des vergrößerten Kopfes (66) eine sich nach hinten und nach innen verjüngende Fläche (68) umfasst.

10. Einspritzvorrichtung nach einem der vorhergehenden Ansprüche, wobei der Auslöser (70) einen in Axialrichtung beweglichen Knopf umfasst, der an der Rückseite der Einspritzvorrichtung (1) angeordnet ist und eine nach hinten zeigende Fläche zum Eingriff durch einen Benutzer und eine entgegengesetzte nach vorn zeigende Fläche umfasst, und wobei die nach vorn zeigende Fläche dafür angeordnet ist, den hinteren Abschnitt des Kolbens in Eingriff zu nehmen, wenn der Auslöser von der vorbereiteten Stellung zu der Freigabestellung bewegt wird, um die nach vorn vorspannende Kraft des Betätigungselements zu verstärken, um die Rückhaltekraft des Klinkenelements zu überwinden.

11. Einspritzvorrichtung nach Anspruch 10, wobei der Auslöser derart angeordnet ist, dass ein Teil der Betätigungskraft, die durch den Benutzer an dem Auslöser (70) bereitgestellt wird, unmittelbar in eine Vorwärtskraft auf den Kolben (60) übertragen wird.

12. Einspritzvorrichtung nach Anspruch 10 oder 11, wobei die nach vorn zeigende Fläche mit einem sich nach innen erstreckenden Vorsprung (79) zum In- Eingriff-Nehmen einer hinteren Spitze des Kolbens versehen ist.

13. Einspritzvorrichtung nach Anspruch 12, wobei die Spitze des Kolbens mit einer Aussparung oder Vertiefung (69) versehen ist, die dafür bemessen und geformt ist, den Vorsprung (79) aufzunehmen.

14. Einspritzvorrichtung nach einem der vorhergehenden Ansprüche, wobei das Klinkenelement (50) integral mit dem Gehäuse geformt ist.

15. Einspritzvorrichtung nach einem der vorhergehenden Ansprüche, wobei das Klinkenelement (50) ein in Radialrichtung segmentiertes zylindrisches Element umfasst.

## Revendications

1. Dispositif d'injection (1), comprenant :
un piston plongeur (60) pour exprimer un médicament d'une seringue (20), le piston plongeur comportant une tête élargie (66) au niveau d'une extrémité arrière ;
un mécanisme d'actionnement agencé en service pour fournir une force de sollicitation pour pousser le piston plongeur (60) d'une première position arrière vers une deuxième position avant pour exprimer une dose de médicament ;
un élément de verrou (50) comprenant une ouverture (54) à travers laquelle l'extrémité arrière du piston plongeur s'étend dans sa position arrière, de sorte à être retenu contre la force du mécanisme d'actionnement ; et
un déclencheur (70) pour dégager le piston plongeur (60) de l'élément de verrou (50);
l'un de l'élément de verrou et du déclencheur comporte un élément faisant saillie de manière sensiblement radiale (56), qui est agencé pour s'engager dans une surface opposée (78) de l'autre du déclencheur et de l'élément de verrou ; et
**caractérisé en ce que** :
l'élément de verrou (50) est agencé pour se déplacer au moins en partie dans une direction radiale et est segmenté radialement par au moins une paire de fentes opposées à extension axiale (58) pour fournir deux ou plusieurs segments opposés ; et
l'ouverture (54) est fournie dans une paroi transversale (52) au niveau de l'arrière de l'élément de verrou, les fentes (58) se rejoignant au niveau de la paroi transversale (52) et les fentes étant reliées à l'ouverture (54) ;
de sorte que l'ouverture (54) se dilate lorsque l'élément de verrou est déplacé radialement pour dégager la tête élargie (66) du piston plongeur ; et
dans lequel le déclencheur (70) peut être déplacé entre :
une position amorcée, dans laquelle l'élément (56) et la surface opposée (78) empêchent le déplacement radial de l'élément de verrou ; et
une position dégagée, dans laquelle l'élément à saillie radiale (56) se déplace à l'écart de la surface opposée pour permettre le déplacement du verrou.

2. Dispositif d'injection selon la revendication 1, dans lequel au moins une partie du déclencheur (70) est disposée radialement à l'extérieur de l'élément de verrou (50).

3. Dispositif d'injection selon la revendication 2, dans lequel le déclencheur (70) est monté de manière coulissante à l'intérieur d'un boîtier (10) du dispositif d'injection et comprend une partie s'étendant vers l'avant (78) qui est disposée radialement à l'extérieur de l'élément de verrou (50).

4. Dispositif d'injection selon la revendication 3, dans lequel le déclencheur (70) comprend un bouton pouvant être déplacé axialement.

5. Dispositif d'injection selon les revendications 3 ou 4, dans lequel la partie s'étendant vers l'avant (78) entoure sensiblement l'élément de verrou (50).

6. Dispositif d'injection selon l'une quelconque des revendications précédentes, dans lequel l'élément faisant saillie vers l'extérieur comprend au moins un bossage faisant saillie radialement vers l'extérieur (56) fourni sur une surface externe de l'élément de verrou, et la surface opposée comprend une surface interne du déclencheur (70) contre laquelle le bossage (56) bute lorsque le déclencheur se trouve dans la position amorcée.

7. Dispositif d'injection selon la revendication 6, dans lequel le déclencheur (70) comprend au moins une découpe (76) dimensionnée et formée de sorte à recevoir l'au moins un bossage faisant saillie vers l'extérieur (56), et dans lequel la découpe est déplacée en alignement avec le bossage lorsque le déclencheur se trouve dans la position dégagée.

8. Dispositif d'injection selon l'une quelconque des revendications précédentes, dans lequel la tête élargie (66) comprend une surface effilée vers l'arrière et vers l'extérieur (67) sollicitant les parties flexibles (52) de l'élément de verrou (50) vers l'extérieur lorsque le piston plongeur (60) passe à travers l'ouverture (54).

9. Dispositif d'injection selon la revendication 8, dans lequel une partie arrière de la tête élargie (66) comprend une surface effilée vers l'arrière et vers l'intérieur (68).

10. Dispositif d'injection selon l'une quelconque des revendications précédentes, dans lequel le déclencheur (70) comprend un bouton à déplacement axial disposé au niveau de l'arrière du dispositif d'injection (1) et comprenant une surface orientée vers l'arrière destinée à être engagée par un utilisateur, et une surface opposée orientée vers l'avant, et dans lequel la surface orientée vers l'avant est agencée pour s'engager dans la partie arrière du piston plongeur lorsque le déclencheur est déplacé de la position amorcée vers la position dégagée pour renforcer le force de sollicitation vers l'avant de l'élément d'actionnement, pour surmonter la force de retenue de l'élément de verrou.

11. Dispositif d'injection selon la revendication 10, dans lequel le déclencheur est agencé de sorte qu'une partie de la force d'actionnement appliquée par l'utilisateur sur le déclencheur (70) est transférée directement en une force vers l'avant appliquée sur le piston plongeur (60).

12. Dispositif d'injection selon les revendications 10 ou 11, dans lequel la surface orientée vers l'avant comporte une saillie s'étendant vers l'intérieur (79) destinée à s'engager dans une pointe arrière du piston plongeur.

13. Dispositif d'injection selon la revendication 12, dans lequel la pointe du piston plongeur comporte un évidement ou un creux (69) dimensionné et formé de sorte à recevoir la saillie (79).

14. Dispositif d'injection selon l'une quelconque des revendications précédentes, dans lequel l'élément de verrou (50) est formé d'une seule pièce avec le boîtier.

15. Dispositif d'injection selon l'une quelconque des revendications précédentes, dans lequel l'élément de verrou (50) comprend un élément cylindrique à segmentation radiale.
